(19) Europäisches Patentamt
European Patent Office
Office européen des brevets

(11) Publication number: **0 234 242 B1**

(12) # EUROPEAN PATENT SPECIFICATION

(45) Date of publication of patent specification: **07.08.91**

(51) Int. Cl.5: **C07D 249/08**, C07D 233/60, A01N 43/653, A01N 43/50, C07C 67/343, C07C 253/00

(21) Application number: **87100614.4**

(22) Date of filing: **19.01.87**

The file contains technical information submitted after the application was filed and not included in this specification

(54) **Fungicidal azolyl-derivatives.**

(30) Priority: **23.01.86 IT 1916986**

(43) Date of publication of application:
**02.09.87 Bulletin 87/36**

(45) Publication of the grant of the patent:
**07.08.91 Bulletin 91/32**

(84) Designated Contracting States:
**AT BE CH DE ES FR GB GR LI NL SE**

(56) References cited:
**EP-A- 0 110 570**
**EP-A- 0 122 452**
**EP-A- 0 145 294**
**GB-A- 1 589 852**

(73) Proprietor: **MONTEDIPE S.r.l.**
**16, Piazza della Repubblica**
**I-20124 Milan(IT)**

(72) Inventor: **Colle, Roberto Dr.**
**821, Residenza Parco Milano 3**
**I-20089 Basiglio (MI)(IT)**
Inventor: **Corda, Francesco**
**13, via Teodosio**
**I-20131 Milano(IT)**
Inventor: **Camaggi, Giovanni Dr.**
**32, via Defendente**
**I-20075 Lodi (MI)(IT)**
Inventor: **Gozzo, Franco Dr.**
**36/b, via Pascoli**
**I-20097 S. Donato Milanese (MI)(IT)**
Inventor: **Mirenna, Luigi**
**4, via Gamboloita**
**I-20139 Milano(IT)**
Inventor: **Garavaglia, Carlo**
**7, piazza Vittoria**
**I-20012 Cuggiono (MI)(IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

**EP 0 234 242 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to azolyl-derivatives endowed with high fungicidal activity, to the process for their preparation, and to their related use in the agrarian field.

EP-A-110 570 is directed to fungicidal compounds of general formula

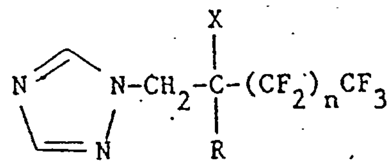

where R is a phenyl group optionally substituted by 1 to 3 substituents each independently selected from F, Cl, Br, I, $CF_3$, $C_1$-$C_4$ alkyl and $C_1$-$C_4$ alkoxy, or R is a 5-chloropyrid-2-yl group; X is F, Cl or Br; and n is zero or an integer of from 1 to 5; and their pharmaceutically and agriculturally acceptable salts.

From GB-A-1,589,852 there are known 1-(2-aryl-2-R-ethyl)1H-1,2,4-triazoles having the general formula:

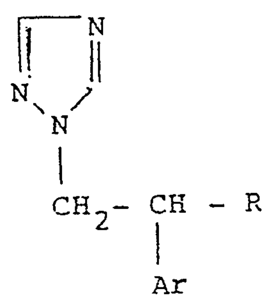

wherein R is alkyl, cycloalkyl, cycloalkylalkyl, alkenyl, arylalkyl, aryloxyalkyl. By the term "alkyl", the radical of an aliphatic hydrocarbon containing from 1 to 10 carbon atoms is meant.

There has now been found a class of 1-(2-aryl-2-R-ethyl)1H-azoles, wherein the radical R has meanings different from those of the latter prior art, endowed with higher fungicidal activity.

An object of the present invention, therefore, are compounds, having the general formula:

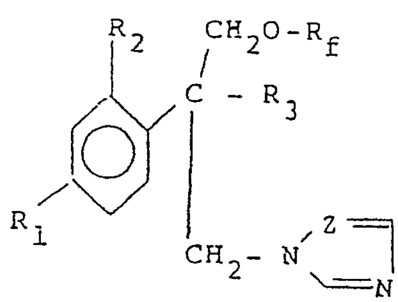

(I)

and salts and metal complexes thereof, wherein:

Z represents CH or N;

$R_1$ is selected from chlorine, bromine, fluorine, $CF_3$, phenyl, $C_1$-$C_2$-alkoxy, $C_1$-$C_2$-haloalkoxy, $C_1$-$C_2$-alkyl thio, $C_1$-$C_2$-haloalkylthio, wherein the halogen is Cl,Br, F;

$R_2$ is H, fluorine, chlorine or bromine;

$R_3$ represents H, $CH_3$, CN, or F;

$R_f$ is selected from polyfluoroalkyl, polyfluoroalkenyl and polyfluoroalkynyl radicals containing up to 4 carbon atoms, at least 2 F atoms and, optionally, other halogen atoms selected from Cl and Br.

Examples of groups $R_f$ which can be present, according to the present invention, and which are indicated for non-limitative purposes are:

alkyl : $-CHF-CF_3$, $-CHBr-CF_3$. $-CHCl-CF_3$, $-CH_2-CF_3$, $-CF_2-CF_2H$, $-CF_2-CFH-CF_3$;

alkenyl : $-CF=CF_2$, $-CF=CF-CF_3$. $-CH=CF-CF_3$, $-CH=CCl-CF_3$, $-CH=CBr-CF_3$, $-CH=C(CF_3)_2$;
alkynyl : $-C\equiv C-CF_3$.

The compounds according to the present invention may have one or more chiral centre(s).

These compounds are generally obtained as racemic mixtures. These mixtures can be separated into the individual enantiomers by methods known from the literature.

Both the individual enantiomers and the possible diastereoisomers or geometric isomers, generated by several chiral centers or by possible double bonds, are an object of the present invention.

As already indicated above, objects of the present invention are also:

- the salts of the compounds having general formula (I) derived from an inorganic acid, such as a hydrogen halide, e.g., hydroiodic, hydrobromic, hydrochloric acid; sulphuric, nitric, thiocyanic and phosphoric acid; or from an organic acid, such as acetic, propanoic, ethanedioic, propanedioic, benzoic, methanesulphonic, 4-methylbenzenesulphonic acid, etc.;
- the metal complexes obtained by the complexation reaction of the compounds of formula (I) with an organic or inorganic salt of a metal, such as halides, nitrates, sulphates, phosphates of, e.g., copper, manganese, zinc or iron.

Examples of compounds of general formula (I) according to the present invention are reported in Table 1.

Table 1

| Compound No. | $R_1$ | $R_2$ | $R_3$ | Z | $R_f$ | m.p. |
|---|---|---|---|---|---|---|
| 1 | Cl | H | H | N | $-CF_2-CF_2H$ | oil |
| 2 | Cl | Cl | H | N | $-CF_2-CF_2H$ | oil |
| 3 | Cl | Cl | H | N | $-CF_2-CFH-CF_3$ | oil |
| 4 | Cl | Cl | H | N | $-CF=CF-CF_3$ | oil |

The compounds having formula (I) can be obtained by employing various processes, according to the nature of group $R_3$. These processes are briefly described below.

One possible process for the preparation of compounds of formula (I) consists in adding an alcohol of formula

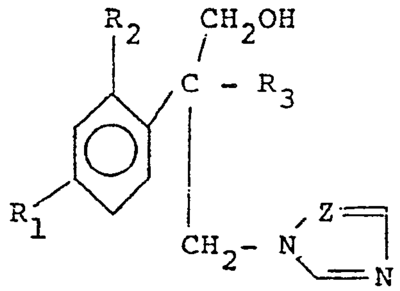

(II)

wherein $R_1$, $R_2$, $R_3$ and Z have the meanings specified above, to a 1,1-difluoroolefin having the formula:

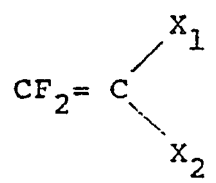

wherein $X_1$ is Cl, F, $CF_3$ and $X_2$ is F, $CF_3$, in an aprotic dipolar solvent, such as, e.g., dimethylformamide, or in an alcoholic solvent, such as, e.g., tert.butanol, in the presence of catalytic or stoichiometric amounts of a strong base, such as, e.g., sodium hydride or potassium tert.butoxide, at temperatures of from 20°C to 100°C, to yield the compounds having formula:

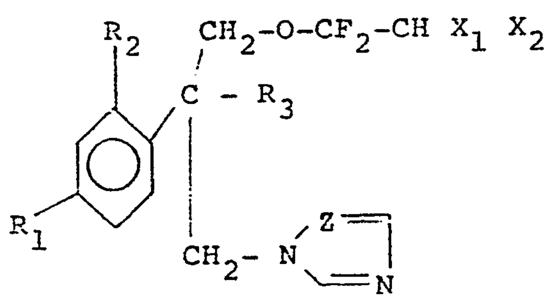

(Ia)

By a dehydrofluorination reaction, which can also take place spontaneously during the above-described reaction, a double bond at the carbon atom in the alpha-position of group $R_f$ can be introduced.
In their turn, the intermediate alcohols of formula (II) can be prepared according to various methods.

a) One possible process for preparing the intermediate alcohols having formula (II) wherein $R_3$ is H, $CH_3$, F, consists in reacting an ester of formula:

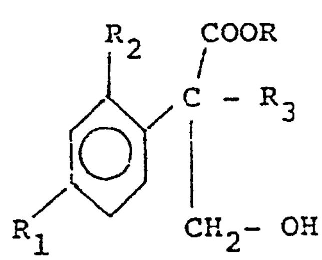

(V)

$CH_3$ or $C_2H_5$,

wherein R is $CH_3$ or $C_2H_5$, known, or obtainable by known methods (Schwenker, Preuntzell, Gassner and Gerber - Chem. Ber., 99 (1966), 2407), with a halogenating agent such as, for example, $SOCl_2$, $POCl_3$,

$PCl_5$, $PBr_3$ and $PBr_5$, or a mesylating agent such as, e.g., methanesulphonyl chloride; or with a tosylating agent, such as., e.g., 4-methylphenylsulphonyl chloride, according to known modalities; in subsequently condensing the intermediate obtained, having formula:

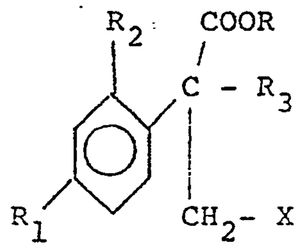

(IV)

wherein X represents halogen, a mesyl radical or a tosyl radical, with the alkali-metal salt of an azole of formula:

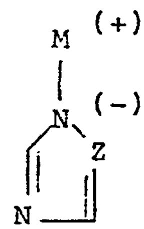

wherein M is an alkali metal and Z has the meaning given above, in an aprotic dipolar solvent, such as DMSO, DMF or acetone, at temperatures of from 20°C to the reflux temperatures of the solvents; and in finally subjecting the obtained intermediate compound, having the formula:

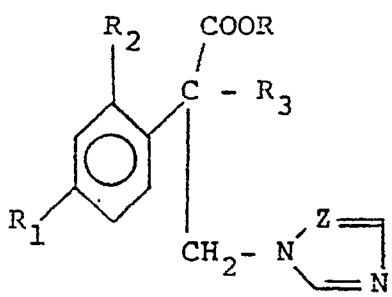

(III)

to reduction, by means of metal hydrides as, e.g., $LiAlH_4$, in ether solvents, such as ethyl ether or THF. The intermediate esters of formula (III) wherein $R_3$ is H, can be prepared, according to an alternative route, by means of the addition of an azole of formula:

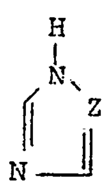

to a known compound of formula:

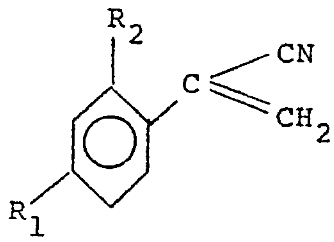

(IVb)

(known, for example, from Colonge, Dreux et Regeand, Bull. Soc. Chim. Fr., 1959, 1244), in apolar solvents such as, e.g., toluene or benzene, in the presence of catalytic amounts of an organic base such as, e.g., triethylamine, at boiling temperature, or in an alcoholic solvent, in the presence of catalytic or stoichiometric amounts of an alkali-metal base, such as, e.g., NaOH or KOH, at the reflux temperature of the solvent, and in subsequently converting -CN group of the intermediates obtained, of formula:

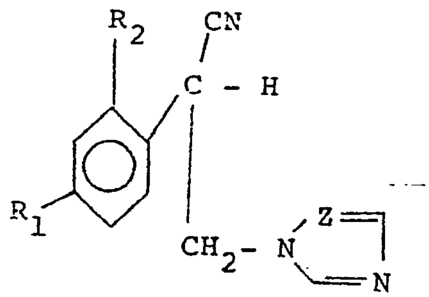

(IIIb)

into a -COOR group, by treatment with mineral acids, such as, e.g., gaseous HCl, $H_2SO_4$, in alcoholic solvents, at temperatures of from 0° C to the boiling temperature of the solvent. By operating in this manner, the esters (III) wherein $R_3$ = H can be converted by reduction, as indicated above, to the alcohols (II), wherein $R_3$ = H.

b) Another route for the preparation of the intermediate alcohols having formula (II) wherein $R_3$ is -CN consists in reacting the above intermediate of formula (IIIb) with paraformaldehyde or trioxymethylene, in aprotic dipolar solvent, such as, e.g., DMSO or DMF, in the presence of catalytic amounts of a strong base such as, e.g., sodium methoxide or sodium ethoxide, KOH, NaOH or sodium hydride, at temperatures comprised between room temperature and 100° C.

The compounds having general formula (I) are endowed with fungicidal activity particularly high against phytopathogenous fungi which attack cultivations of cereals. Cucurbitaceae, grapevines and fruit-trees.

Examples of plant diseases which can be controlled by the compounds of the present invention are the following:

- Erysiphe graminis on cereals;
- Sphaerotheca fuliginea of Cucurbitaceae (e.g., of cucumber)
- Puccinia on cereals
- Septoria on cereals
- Helminthosporium on cereals
- Rhyncosporium on cereals
- Podosphaera leucotricha on apple-trees
- Uncinula necator on grapevines
- Venturia inaequalis on apple-tree
- Piricularia oryzae on rice
- Botrytis cinerea
- Fusarium on cereals

and still further diseases.

The compounds having formula (I) are furthermore endowed with other positive characteristics, such as a fungicidal activity both curative and preventive in character, as well as a complete tolerability by the plants to be protected against the fungal infection.

Besides the high fungicidal activity with preventive and curative application, the compounds of formula

(I) are characterized by systemic properties.

These properties allow the products to enter into the vascular systems of plants, and act in sites (e.g., leaves) even far remote from those to which they are applied (e.g., roots).

For practical use in agriculture, having available fungicidal compositions containing one or more compounds of formula (I) as the active substance is often useful.

The application of these composition can be carried out on any part of the plants, e.g., on leaves, stems, limbs and roots, or on the seeds thereof, before the sowing, or also on the soil the plant is growing on. Compositions can be used, which have the form of dry powders, wettable powders, emulsifiable concentrates, pastes, granulates, solutions, suspensions, and so forth: the selection of the type of composition depends on the specific use. The compositions may be prepared in known way, e.g., by diluting or dissolving the active substance with a solvent means and/or a solid diluent, optionally in the presence of surface-active agents. As solid diluents, or carriers, there can be used: silica, kaolin, bentonite, talc, fossil flour, dolomite, calcium carbonate, magnesia, gypsum, clays, synthetic silicates, attapulgite and sepiolite. As liquid diluents, besides of course water, various types of solvents can be used such as, e.g., aromatic solvents (benzene, xylenes or mixtures of alkylbenzenes), chloroaromatic solvents (chlorobenzene), paraffins (petroleum fractions), alcohols (methanol, propanol, butanol), amines, amides (dimethylformamide), ketones (cyclohexanone, acetophenone, isophorone, ethyl-amyl-ketone) and esters (isobutyl acetate). Examples of suitable surfactants are: sodium, calcium or triethanolamine salts of alkylsulphates, alkylsulphonates, alkyl-arylsulphonates, polyethoxylated alkylphenols, fatty alcohols condensed with ethylene oxide, polyoxyethylated fatty acids, polyoxyethylated sorbitol esters, polyoxyethylated fats and lignine sulphonates. The compositions can also contain special additives for particular purposes, e.g., such adhesive-properties-conferring agents as gum arabic, polyvinyl alcohol and polyvinylpyrrolidone.

If desired, also other compatible substances active as fungicides, phytomedicines, phytoregulators, herbicides, insecticides, fertilizers can be added to the compositions of the present invention.

The concentration of active substance in said compositions can vary within a wide range, depending on the active compound, the cultivation, the pathogenic agent, the environmental conditions and the type of formulation adopted. In general, the concentration of active substance varies from 0.1 to 95%, preferably from 0.5 to 90% by weight.

The following examples illustrate the present invention.

EXAMPLE 1

Preparation of 1-(1H-1,2,4-triazol-1-yl)-2-(4-chlorophenyl)-3-(1,1,2,2-tetrafluoroethoxy)-propane (Compound No. 1)

0.4 g of NaH in oil suspension at 55% is dispersed in 10 ml of anhydrous DMF under a nitrogen atmosphere. At room temperature, 2.4 g of 2-(4-chlorophenyl)-3-(1H-1,2,4-triazol-1-yl)-propanol dissolved in 10 ml of anhydrous DMF, are added. When the reaction is complete (after about 30 minutes), the reaction mixture is cooled to 0° C, and tetrafluoroethylene is added, the flow rate thereof being adjusted in a manner such that the reaction temperature does not exceed 30° C.

At the end of heat evolution, the temperature is allowed to rise again to room temperature.

The reaction mixture is poured into water, and is extracted with dichloromethane. The organic extract is washed with water, dried over $Na_2SO_4$ and evaporated under vacuum. The residual oil (2.9 g) is purified over silica gel, using 8:2 n-hexane + diethylether as the eluent. 0.5 g of colourless viscous oil is obtained, which is identified as title compound on the basis of the following spectroscopic data.

- I.R. ($\nu$, $cm^{-1}$): 680, 1120, 1210, 1275, 1500.
- $^1$H-N.M.R. (200 MHz) in $CDCl_3$,

| | | |
|---|---|---|
| δ= | 3.56 | (quint., 1 H) |
| | 4.11 | (m, 2 H) |
| | 4.42 | (2dd, 2 H) |
| | 5.667 | (tt, 1 H) |
| | 6.945-7.013; 7.181-7.249 | (2 m, 4 H) |
| | 7.681 | (s, 1 H) |
| | 7.853 | (s, 1 H) |

EXAMPLE 2

Preparation of 2-(4-chlorophenyl)-3-(1H-1,2,4-triazol-1-yl)-propanol

1.3 g of $LiAlH_4$ is suspended in 170 ml of anhydrous diethyl ether under a $N_2$ atmosphere. Within one hour, 8 g of methyl α-(1H-1,2,4-triazol-1-ylmethyl)-4-chlorophenylacetate are introduced. The reaction is exothermic, and proceeds with solvent reflux. At the end of heat evolution, 10 ml of methanol in 50 ml of diethyl ether are added to decompose the excess hydride.

The reaction mixture is filtered over celite and from the filtrate, after concentration, 6.8 g of solid product are obtained which are washed with a few ml of n-hexane - acetone mixture, to obtain 5.3 g of a white solid (m.p. 137-9 °C) having the structure indicated above.

- I.R. (ν, $cm^{-1}$) in oil: 830, 1018, 1065, 1140, 1284, 3115, 3210.

EXAMPLE 3

Preparation of methyl α-(1H-1,2,4-triazol-1-yl)-4-chlorophenylacetate

A suspension of 3.2 g of 1,2,4-triazole and 8.3 g of $K_2CO_3$ in 300 ml of anhydrous acetone is refluxed over 1 hour. After cooling to 10 °C, 14 g of methyl α-(methanesulphonyloxymethyl)-4-chlorophenyl-acetate are added. The temperature is then allowed to spontaneously rise to room temperature, and the reaction mixture is stirred for one hour, is filtered over fritted glass and the filtrate is concentrated under vacuum, thereby obtaining 13 g of a crude solid product, which is suspended with 10 ml of 1:1 n-hexane / ethanol mixture.

10.8 g of white solid (m.p. 92-3 °C), having the structure indicated above are thus obtained.

- I.R. (ζ, $cm^{-1}$) in oil: 840, 1019, 1092, 1142, 1225, 1743.

EXAMPLE 4

Preparation of methyl α-(methanesulphonyloxymethyl)-4-chlorophenyl-acetate

To a solution of 5 g of methyl α-(hydroxymethyl)-4-chlorophenylacetate and 3.5 g of methanesulphonyl chloride in 30 ml of anhydrous diethyl ether, cooled at 0 °C, a solution of 2.6 g of triethylamine in 10 ml of anhydrous diethyl ether is added dropwise. The temperature is allowed to rise again to room temperature, the reaction mixture is poured into water and is extracted with diethyl ether. The ether solution, washed with water, and dried over $Na_2SO_4$, is concentrated under vacuum, to yield 7.2 g of crude product. By crystallization thereof from 5 ml of ethanol, 5.4 g of white solid product (m.p. 77-8 °C), having the structure indicated above, are obtained.

- I.R. (ν, $cm^{-1}$): 1100, 1180, 1210, 1492, 1725.

EXAMPLE 5

Preparation of 1-(1H-1,2,4-triazol-1-yl)-2-(2,4-dichlorophenyl)-3-(1,1,2,2,-tetrafluoroethoxy)-propane (Compound No. 2)

This compound is prepared by a process similar to that disclosed in Example 1, by starting from 2-(2,4-dichlorophenyl)-3-(1H-1,2,4-triazol-1-yl)-propanol, prepared in turn by a process similar to that described in Examples 4, 3 and 2.

Compound No. 2 is isolated as colourless viscous oil and is characterized by the following spectroscopic data.

- I.R. ($\nu$, $cm^{-1}$): 683, 1120, 1210, 1278, 1478, 1505.
- $^1$H-N.M.R. (60 MHz) in $CCl_4$,

| | |
|---|---|
| $\delta$ = 4 - 4.7 | (m, 5 H) |
| 5.72 | (tt, 1 H) |
| 7 - 7.6 | (m, 3 H) |
| 7.74 | (s, 1 H) |
| 7.8 | (s, 1 H) |

EXAMPLE 6

Preparation of methyl 2-(4-chlorophenyl)-4-bromo-3,4,5,5,5-pentafluoropent-2-enoate

0.8 g of sodium hydride in oil suspension at 55% is dispersed in 10 ml of anhydrous DMF under a $N_2$ atmosphere.

At room temperature, 3 g of methyl 4-chlorophenyl-acetate dissolved in 10 ml of anhydrous DMF,are added. When the reaction is complete (after about 30 minutes), the reaction mixture is added, dropwise and under a $N_2$ atmosphere, to a solution of 5 g of 1,2-dibromo-1,1,2,3,3,3-hexafluoropropane in 10 ml of anhydrous DMF. After 1 hour at room temperature, the reaction mixture is poured into water, and is extracted with dichloromethane. The organic extract is washed with water up to neutral pH, dried over $Na_2SO_4$ and evaporated under vacuum to yield 4 g of crude product. This is purified by chromatography over silica gel, using 95:5 n-hexane / diethylether as the eluent. 1.5 g of slightly yellow liquid are obtained, having the structure indicated above, according to the following spectroscopic data:

- I.R. ($\nu$, $cm^{-1}$): 900, 1125, 1220, 1282, 1492, 1594, 1664, 1740.
- $^1$H-N.M.R (60 MHz) in $CCl_4$,

| | |
|---|---|
| $\delta$ = 3.82 | (s, 3 H) |
| 7.43 | (s broad, 4 H) |

EXAMPLE 7

Preparation of 1-(1H-1,2,4-triazol-1-yl)-2-(2,4-dichlorophenyl)-3-(1,1,2,3,3,3-hexafluoropropyloxy)-propane (Compound No. 3) and of 1-(1H-1,2,4-triazol-1-yl)-2-(2,4-dichlorophenyl)-3-(1,1,2,3,3,3-hexafluoropropenyloxy)-propane (Compound No. 4)

The above mentioned compounds are obtained as a mixture, by starting from 2-(2,4-dichlorophenyl)-3-(1H-1,2,4-triazol-1-yl)propanol and hexafluoropropene, and by a process analogous to that disclosed in Example 1.

The compounds are isolated as colourless viscous oils, and characterized by the following spectroscopic data:

Compound No. 3:

- I.R. ($\nu$ , $cm^{-1}$) : 1760, 1590, 1510, 1478, 1280, 1190, 1040.
- $^1$H-N.M.R. (60 MHz) in $CCl_4$,

| | |
|---|---|
| $\delta$ = 7.75 | (s, 1 H) |
| 7.7 | (s, 1 H) |
| 7.50-6.80 | (m, 3 H) |
| 5.25 | (m. 0,5) |
| 4.40-4.45 | (d, 2 H) |
| 4.20 | (s broad, 2 H) |
| 4.7 -3.8 | (m, 1.5 H) |

Compound No. 4:

- The I.R. spectrum is equal to that of Compound No. 3, with the exception of the band at 1760 $cm^{-1}$, which is absent.
- $^1$H-N.M.R. (60 MHz) in $CCl_4$,

| | |
|---|---|
| $\delta$ = 7.75 | ( s, 1 H) |
| 7.70 | (s, 1 H) |
| 7.50-6.80 | (m, 3 H) |
| 4.40-4.45 | (d, 2 H) |
| 4.20 | (s broad, 2 H) |
| 4.55-3.80 | (m, 1 H). |

EXAMPLE 8

Preparation of 1-(1,2,4-triazol-1-yl)-2-(4-chlorophenyl)-2-(1,1,2,2-tetrafluoroethoxy)-methyl-propane (Compound No.5).

0,1 g of potassium tert.-butoxide is added, under a nitrogen atmosphere at -10°C, to 1.9 g of 2-(4-chlorophenyl)-2-methyl-3-(1,2,4-triazol-1-yl)-1-hydroxy-propane dissolved in 6.5 ml of anhydrous THF, 13 ml of anhydrous DMSO and 13 ml of anhydrous tert.-butanol.
The apparatus is then evacuated and tetrafluoroethylene is introduced by maintaining the reaction mass under this gas atmosphere over night, at room temperature.
The reaction mixture then is poured into water, and is extracted with ethyl acetate. The extract is washed with water, dried over $Na_2SO_4$ and evaporated; the crude product obtained is purified by chromatography over silica gel, using n-hexane-ethyl acetate 1:1 as eluent. 1 g of an oil is isolated, having the structure indicated above, according to the following spectroscopic data:

- I.R. ($cm^{-1}$) 1580, 1280, 1210, 1120.
- $^1$H-NMR (60 MHz) TMS in $CDCl_3$,

$$\delta = 1.30\ (s,\ 3\ H),\ 4.00\ (s\ broad,\ 2\ H);\ 4.25\ (s\ broad,\ 2\ H);\ 5.55\ (tt,\ 1\ H);\ 6.80\text{-}7.20\ (m,\ 4\ H);\ 7.35\ (s,\ 1\ H);\ 7.55\ (s,\ 1\ H).$$

Similar to Compound No. 5, 1-(1,2,4-triazol-1-yl)-2-(2,4-dichlorophenyl)-2-(1,1,2,2-tetrafluoroethoxy)-methyl-propane is prepared.
The compound is characterized by the following spectroscopic data.
- $^1$H-NMR (60 MHz) TMS in $CDCl_3$.

$$\delta = 1.35\ (s,\ 3\ H);\ 3.90\ (s\ broad,\ 2\ H);\ 4.05\ (s\ broad,\ 2\ H);\ 5.50\ (tt,\ 1\ H);\ 6.80\text{-}7.20\ (m,\ 3\ H);\ 7.45\ (s,\ 1\ H);\ 7.60\ (s,\ 1\ H).$$

EXAMPLE 9

Determination of the fungicidal activity against cucumber oidium (Sphaerotheca fuliginea(schlech) Salmon)

Preventive activity:

Cucumber plants, cv. Marketer, grown in pot in conditioned environment, were sprinkled on the lower faces of their leaves with the test compounds in water-acetone solution at 20% (v/v) of acetone. The plants were subsequently kept in conditioned environment for 1 day, and then sprinkled on the upper face of the leaves with an aqueous suspension of conidia of Sphaerotheca fuliginea (200,000 conidia per ml). The plants were then placed again in conditioned environment.

At the end of the incubation period of the fungus (8 days), the severity of the infection was evaluated, and given a rating based on an evaluation scale ranging from 100 (= healthy plant) to 0 (= completely infected plant).

Curative activity:

Plants of cucumber cv. Marketer, grown in pot in conditioned environment, were sprayed on the upper face of the leaves with an aqueous solution of conidia of Sphaerotheca fuliginea (200,000 conidia per ml). After 24 hours from the infection, the plants were treated with the test compounds in water-acetone solution at 20% (v/v of acetone), by spraying both faces of their leaves.

At the end of the incubation period of the fungus (8 days), during which the plants were kept in a suitably conditioned environment, the severity of the infection was evaluated and given a rating based on an evaluation scale ranging from 100 (= healthy plant) to 0 (= completely infected plant).

The results are reported in Table 2.

EXAMPLE 10

Determination of the fungicidal activity against the oidium of wheat (Erysiphe graminis D.C.)

Preventive activity:

The leaves of wheat, cv. Irnerio, grown in pot in conditioned environment, were treated by sprinkling both faces with the test compounds in water-acetone solution at 20% (v/v) of acetone.

After one day in conditioned environment, the plants were sprinkled on both faces of their leaves with an aqueous suspension of Erysiphe graminis (200,000 conidia per ml). After 24 hours in a hunidity-saturated environment, at 21 °C, the plants were kept in a conditioned environment for the incubation of the fungus.

At the end of said incubation period (12 days), the severity of the infection was evaluated visually and was given a rating based on an evaluation scale ranging from 100 (= healthy plant) to 0 (= completely infected plant).

Curative activity:

The leaves of wheat,cv. Irnerio, grown in pot in conditioner environment, were sprayed on both faces with an aqueous suspension of Erysiphe graminis (200,000 conidia per ml). After 24 hours in a humidity-saturated environment, at 21 °C, the leaves were treated with the test compounds in water-acetone solution at 20% (v/v)of acetone, by spraying both faces.

At the end of the incubation period (12 days), the severity of the infection was visually evaluated, and was given a rating based on an evaluation scale going from 100 (= healthy plant) to 0 (= completely infected plant).

The results are reported in Table 2.

EXAMPLE 11

Determination of the fungicidal activity against the linear blight of wheat (Puccinia graminis Pers.)

Preventive activity:

The leaves of wheat, cv. Irnerio, grown in pot in conditioned environment, were treated by sprinkling both faces with the test compounds in water-acetone solution at 20% (v/v) of acetone. After one day in an environment conditioned at 23 °C and 70% R.H., the plants were sprinkled on both faces of their leaves with a mixture of spores of Puccinia graminis in talc (100 mg of spores per 5 g of talc). After 48 hours in a humidity-saturated environment, at 21 °C, the plants were kept in a conditioned environment for the incubation of the fungus.

At the end of said incubation period (14 days), the severity of the infection was evaluated visually, and was given a rating based on an evaluation scale ranging from 100 (= healthy plant) to 0 (= completely infected plant).

Curative activity:

The leaves of wheat cv. Irnerio, grown in pot in conditioned environment, were sprayed on both faces with a mixture of spores of Puccinia graminis in talc (100 mg of spores/5 g of talc); after 48 hours in a humidity-saturated environment, at 21 °C, the leaves were treated with the test compounds in water-acetone solution at 20% (v/v of acetone), by spraying of both faces.

At the end of the incubation period (14 days), the severity of the infection was visually evaluated, and

was given a rating based on an evaluation scale ranging from 100 (= healthy plant) to 0 (= completely infected plant).

The results are reported in Table 2.

TABLE 2

| Compound No. | Dose g/l | Sphaerotheca fuliginea/cucumber | | Erysiphe graminis trit./wheat | | Puccinia graminis/wheat | |
|---|---|---|---|---|---|---|---|
| | | Preventive Activity | Curative Activity | Preventive Activity | Curative Activity | Preventive Activity | Curative Activity |
| 1 | 0.5 | 100 | 100 | 100 | 100 | 100 | 100 |
| | 0.25 | 100 | 100 | 100 | 100 | 100 | 100 |
| | 0.125 | 100 | 100 | 100 | 100 | 70 | 100 |
| 2 | 0.5 | 100 | 100 | 100 | 100 | 100 | 100 |
| | 0.25 | 100 | 100 | 100 | 100 | 100 | 100 |
| | 0.125 | 100 | 100 | 100 | 100 | 100 | 100 |
| Ref.* | 0.5 | 100 | | 100 | | 50 | |
| | 0.25 | 100 | | 100 | | 0 | |
| | 0.125 | 100 | | 100 | | 0 | |

Ref* corresponds to the reference compound 1-[2-(2,4-dichlorophenyl)pentyl]-1H-1,2,4-triazole, known as Penconazole® (Topas), of GB-A-1,589,852.

EXAMPLE 12

Determination of the Foliar Systemic Activity on Wheat Oidium (Erysiphe graminis d.c.)

The leaves of wheat, cv. Irnerio, grown in pot in conditioned environment, were treated by sprinkling of both faces with the test compounds in water-acetone solution at 20% (v/v) of acetone. Five days after the treatment, the treated leaves and the new leaves grown in the meantime were sprinkled on both faces with an aqueous suspension of Erysiphe graminis (200,000 conidia per ml ). After 24 hours in a humidity-saturated environment, at 21 °C, the plants were kept in a conditioned environment for the incubation of the fungus.

At the end of said incubation period (12 days), the severity of the infection was evaluated visually and was given a rating based on an evaluation scale ranging from 100 (= healthy plant) to 0 (completely infected plant).

The results shown in Table 3 have been obtained.

TABLE 3

Foliar Systemic Activity on Erysiphe graminis

| Compound No. | Dose g/l | Treated Leaves | Untreated Leaves |
|---|---|---|---|
| 2 | 0.0018 | 100 | 100 |
| Penconazole® | 0.0018 | 42 | 14 |
| Propiconazole® | 0.0018 | 40 | 30 |
| Triadimefon® | 0.0018 | 0 | 0 |

- Propiconazole® = l-/2-(2,4-dichlorophenyl)-4-propyl-l,3-dioxolan-2-yl-methyl/-lH-l ,2,4-triazole.
- Triadimefon = l-(4-chlorophenoxy)-3,3-dimethyl-l-(lH-l,2, 4-triazol-l-yl ) -butanone.

EXAMPLE 13

Determination of the Foliar Systemic Activity on linear blight of Wheat (Puccinia graminis Pers.)

The leaves of wheat, cv. Irnerio, grown in pot in conditioned environment, were treated by sprinkling of both faces with the test compounds in water-acetone solution at 20% (v/v) of acetone. Five days after the treatment, the treated leaves and the new leaves grown in the meantime were sprinkled on both faces with a mixture of spores of Puccinia graminis in talc (100 mg of spores/5 g of talc). After 48 hours in a humidity-saturated environment, at 21 °C, the plants were kept in a conditioned environment for the incubation of the fungus.

At the end of said incubation period (14 days), the severity of the infection was evaluated visually and was given a rating based on an evaluation scale ranging from 100 (= healthy plant) to 0 (= completely infected plant).

The results shown in Table 4 have been obtained.

TABLE 4

Foliar Systemic Activity on Puccinia graminis Pers.

| Compound No. | Dose, g/l | Treated Leaves | Untreated Leaves |
|---|---|---|---|
| 2 | 0.5 | 100 | 100 |
| Penconazole® | 0.5 | 20 | 0 |

EXAMPLE 14

Determination of the fungicidal activity on brown-spotting of apple-trees(Venturia inaequalis (cke) Wint)

Preventive activity:

The leaves of apple-trees,cv. Starking, grown in pot in a greenhouse, were treated by sprinkling of both faces with the test compounds in water-acetone solution at 20% (v/v) of acetone. After one day in an environment conditioned at 20° and 70% R.H., the plants were sprinkled on both faces of their leaves with an aqueous suspension of conidia of Venturia inaequalis (200,000 conidia per ml). After 2 days in a humidity-saturated environment, at 21°, the plants were kept in a conditioned environment for the incubation of the fungus.

At the end of said incubation period (14 days), the severity of the infection was evaluated visually and was given a rating based on an evaluation scale ranging from 100 (= healthy plant) to 0 (= completely infected plant).

Curative activity:

The leaves of apple-trees,cv. Starking, grown in pot in a greenhouse, were sprinkled uniformly with an aqueous suspension of conidia of Venturia inaequalis (200,000 conidia per ml). After 2 days in a humidity-saturated environment, said leaves were treated with the tested conpounds in a water-acetone solution at 20% (v/v) of acetone, by sprinkling of both faces.

At the end of the incubation period(l4 days), the severity of the infection was evaluated visually and was given a rating based on an evaluation scale ranging from 100 (= healthy plant) to 0 (= completely infected plant).

The results are reported in Table 5.

TABLE 5

Activity on Venturia inaequalis (Cke) Wint.

| Compound No. | Dose :,g/l | Preventive Activity | Curative Activity |
|---|---|---|---|
| 2 | 0.05 | 100 | 100 |
| Penconazole® | 0.05 | 88 | 88 |
| Propiconazole® | 0.05 | 77 | 75 |

**Claims**

**1.** Compounds having the general formula:

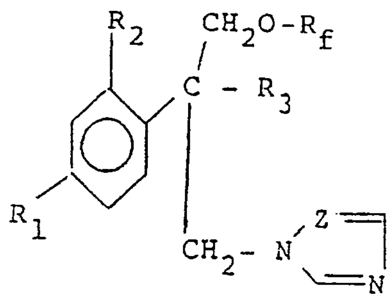

(I)

and salts and metal complexes thereof, wherein:
Z represents CH or N;
$R_1$ is selected from chlorine, bromine, fluorine, $-CF_3$, phenyl, $C_1$-$C_2$-alkoxy, $C_1$-$C_2$-haloalkoxy, $C_1$-$C_2$-akylthio and $C_1$-$C_2$-haloalkylthio wherein the halogen is Cl, Br or F;
$R_2$ is H, fluorine, chlorine or bromine;
$R_3$ represents H, $CH_3$, CN, or F;
$R_f$ is selected from polyfluoroalkyl, polyfluoroalkenyl and polyfluoroalkynyl radicals containing up to 4 carbon atoms, at least 2 F atoms and, optionally, other halogen atoms selected from Cl and Br.

**2.** Compounds according to claim 1, wherein $R_f$ is an alkyl radical containing up to 4 carbon atoms and at least 2 fluorine atoms.

**3.** Compound according to claim 1, which is 1-(1H-1,2,4-triazol-1-yl)-2-(2,4-dichlorophenyl)-3-(1,1,2,2-tetrafluoroethoxy)-propane.

**4.** Process for the preparation of compounds according to claim 2, wherein an alcohol of formula

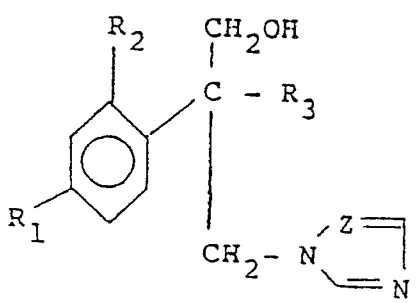

(II)

wherein $R_1$, $R_2$, $R_3$ and Z have the meanings specified in claim 1 is reacted with a 1,1-fluoroolefin having the formula;

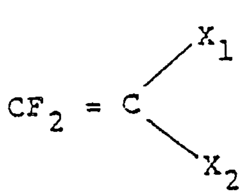

wherein $X_1$ is Cl, F or $CF_3$ and $X_2$ is F or $CF_3$, in a dipolar aprotic or alcoholic solvent, in the presence of catalytic or stoichiometric amounts of a strong base and at temperatures of from 20 °C to 100 °C, to yield the compounds of formula:

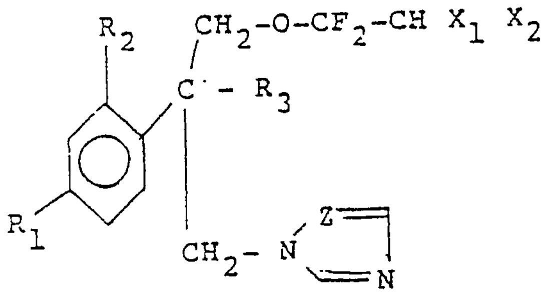

(Ia)

5. Process for the preparation of alcohols of formula:

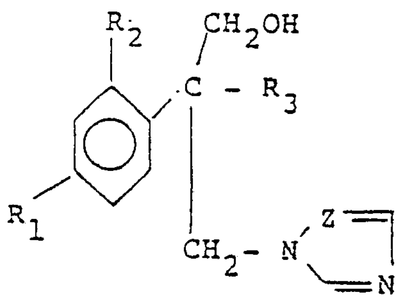

(II)

wherein $R_3$ is H, $CH_3$ or F and $R_1$, $R_2$ and Z have the meanings specified in claim 1, wherein an ester of formula:

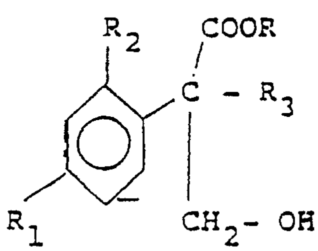

(V)

wherein R is $CH_3$ or $C_2H_5$, is reacted with a halogenating or mesylating or tosylating agent according to known methods, the resulting intermediate of formula:

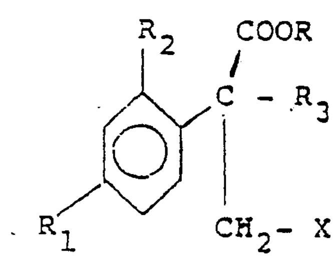

(IV)

wherein X represents halogen, a mesyl radical or a tosyl radical is condensed with an alkali metal salt of an azole having the formula:

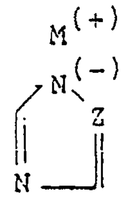

wherein M is an alkali metal and Z has the meaning specified in claim 1, in a dipolar aprotic solvent and at temperatures of from 20° C to the reflux temperature of the reactants, and the compound of formula:

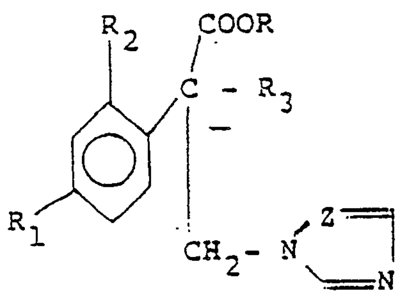

(III)

thus obtained is reduced by metal hydrides in ether solvents.

6. Method for controling or preventing fungal infestations in useful plants, consisting in distributing on the plant, on the seeds or on the surrounding soil, when the fungal infestation is expected or is already in progress, an effective amount of a compound according to any one of claims 1 to 3, as such or in the form of a suitable composition.

7. Antifungal compositions, containing as the active ingredient one or more compounds according to any one of claims 1 to 3, together with a solid or liquid carrier and, optionally, other additives.

**Revendications**

1. Dérivés répondant à la formule générale :

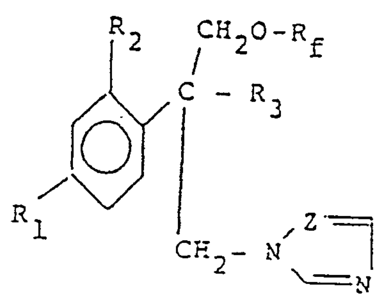

(I)

et leurs sels et complexes métalliques caractérisés en ce que :

z représente CH ou N ;

$R_1$ est choisi entre chlore, brome, fluor, $CF_3$, le radical phényle, un radical alcoxy en $C_1$ à $C_2$, haloalcoxy en $C_1$ à $C_2$, haloalkylthio en $C_1$ à $C_2$, l'atome d'halogène étant Cl, Br ou F ;

$R_2$ représente H, un atome de fluor, de chlore ou de brome ;

$R_3$ représente H, $CH_3$, CN ou F ;

$R_f$ est choisi entre des radicaux polyfluoroalkyles, polyfluoroalkényles et polyfluoroalkynyles renfermant jusqu'à 4 atomes de carbone, au moins 2 atomes de F et éventuellement d'autres atomes d'halogène choisis entre Cl et Br.

**2.** Dérivés selon la revendication 1, caractérisés en ce que $R_f$ représente un radical alkyle contenant jusqu'à 4 atomes de carbone et au moins 2 atomes de fluor.

**3.** Dérivés selon la revendication 1, portant le nom de 1-(1H-1,2,4-triazol-1-yl)-2-(2,4-dichlorophényl)-3-(1,1,2,2-tétrafluoroéthoxy)-propane.

**4.** Procédé de préparation de dérivés selon la revendication 2, caractérisé en ce que l'on fait réagir un alcool de formule :

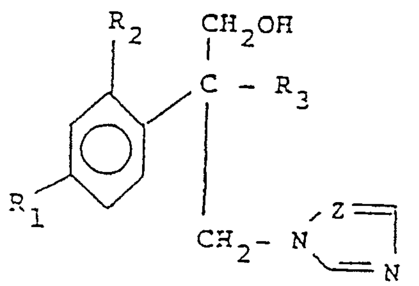

(II)

dans laquelle $R_1$, $R_2$, $R_3$ et Z présentent les significations spécifiées dans la revendication 1, avec une 1,1-fluorooléfine répondant à la formule :

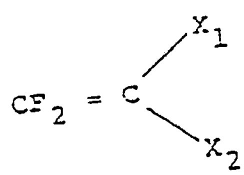

dans laquelle $X_1$ représente Cl, F, $CF_3$ et $X_2$ représente F ou $CF_3$, dans un solvant aprotique ou alcoolique, en présence de quantités catalytiques ou stoechiométriques d'une base forte et à des températures de 20 à 100° C, et l'on obtient le dérivé de formule :

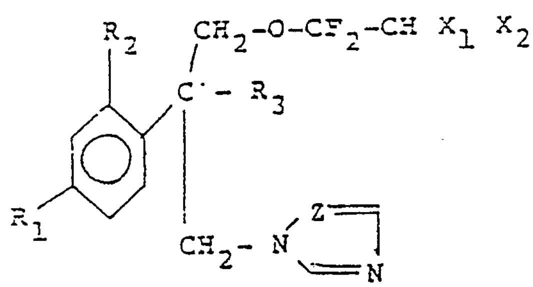

(Ia)

**5.** Procédé de préparation d'alcools de formule :

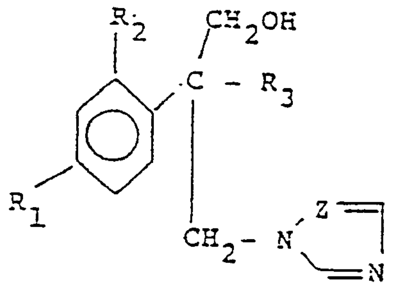

(II)

caractérisé en ce que $R_3$ est H, $CH_3$ ou F et $R_1$, $R_2$ et Z présentent les significations spécifiées dans la revendication 1, caractérisé en ce que l'on fait réagir un ester de formule :

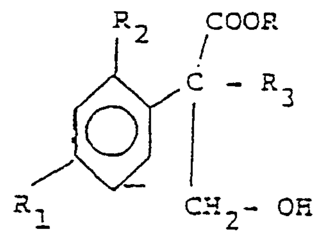

(V)

dans laquelle R représente $CH_3$ ou $C_2H_5$ avec un agent d'halogénation ou de mésylation ou de tosylation par des modes opératoires connus en soi, l'intermédiaire obtenu de formule :

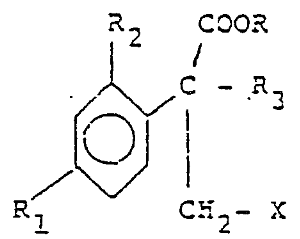

(IV)

dans laquelle X représente un atome d'halogène, un radical mésyle ou un radical tosyle est condensé avec un sel de métal alcalin d'un azole répondant à la formule :

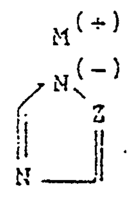

dans laquelle M est un métal alcalin et Z présente la signification spécifiée dans la revendication 1, dans un solvant aprotique dipolaire et à des températures de 20 °C à la température de reflux des réactifs ; et le dérivé de formule :

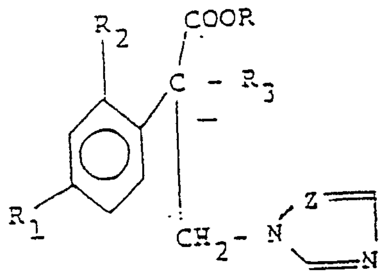

(III)

ainsi obtenu est réduit par des hydrures métalliques dans des solvants à base d'éther.

6. Procédé de contrôle ou de prévention ou d'infestion par les champignons de plantes utiles consistant à distribuer sur la plante, sur les graines ou le sol environnant, lorsque l'on s'attend à une infestation fongique ou qu'elle est déjà en cours, une quantité efficace d'un dérivé selon l'une quelconque des revendications 1 à 3, tel que ou sous la forme d'une composition convenable.

7. Compositions anti-fongiques contenant comme ingrédient actif un ou plusieurs dérivés selon l'une quelconque des revendications 1 à 3 en même temps qu'un support solide ou liquide et, éventuellement, d'autres additifs.

**Patentansprüche**

1. Verbindungen der allgemeinen Formel

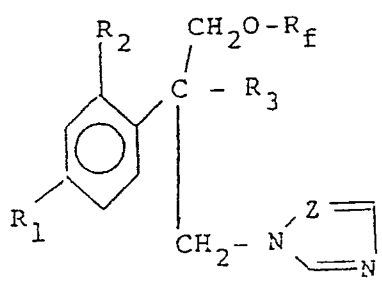

(I)

und deren Salze und Metallkomplexe, worin
Z CH oder N bedeutet;
$R_1$ aus Chlor, Brom, Fluor, $CF_3$, Phenyl, $C_1$-$C_2$-Alkoxy, Cl-$C_2$-Halogenalkoxy, $C_1$-$C_2$-Alkylthio und $C_1$-$C_2$-Halogenalkylthio ausgewählt ist, worin das Halogen Cl, Br oder F ist;
$R_2$ H, Fluor, Chlor oder Brom bedeutet;
$R_3$ H, $CH_3$, CN oder F bedeutet;
$R_f$ aus Polyfluoralkyl-, Polyfluoralkenyl- und Polyfluoralkinylresten ausgewählt ist, die bis zu 4 Kohlenstoffatome, mindestens 2 F-Atome und gegebenenfalls andere Halogenatome, ausgewählt aus Cl und Br, enthalten.

2. Verbindungen gemäß Anspruch 1, in welchen $R_f$ ein Alkylrest mit bis zu 4 Kohlenstoffatomen und mindestens 2 Fluoratomen ist.

3. Verbindung gemäß Anspruch 1, die 1-(1H-1,2,4-Triazol-1-yl)-2-(2,4-dichlorphenyl)-3-(1,1,2,2-tetrafluorethoxy)propan ist.

4. Verfahren zur Herstellung von Verbindungen gemäß Anspruch 2, bei welchem ein Alkohol der Formel

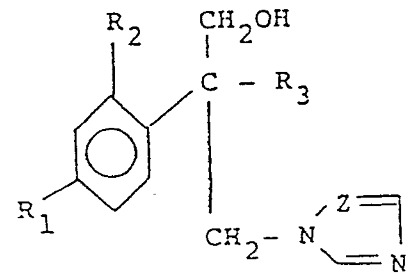

(II)

worin $R_1$, $R_2$, $R_3$ und Z die in Anspruch 1 angegebenen Bedeutungen haben, mit einem 1,1-Fluorolefin der Formel

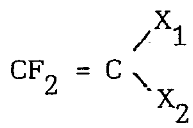

worin $X_1$ Cl, F oder $CF_3$ ist und $X_2$ F oder $CF_3$ ist, in einem dipolaren aprotischen oder alkoholischen Lösungsmittel in Gegenwart katalytischer oder stöchiometrischer Mengen einer starken Base und bei Temperaturen von 20 bis 100° C zur Bildung der Verbindungen der Formel

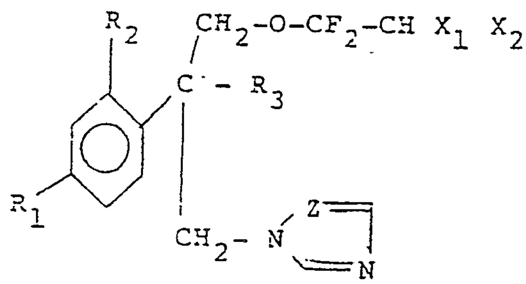

(Ia)

umgesetzt wird.

**5.** Verfahren zur Herstellung von Alkoholen der Formel

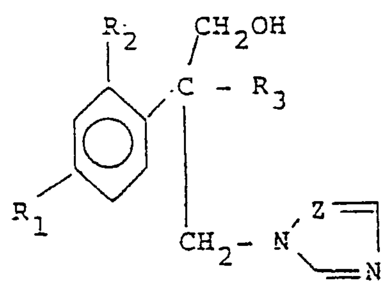

(II)

worin $R_3$ H, $CH_3$ oder F ist und $R_1$, $R_2$ und z die in Anspruch 1 angegebenen Bedeutungen haben, bei dem ein Ester der Formel

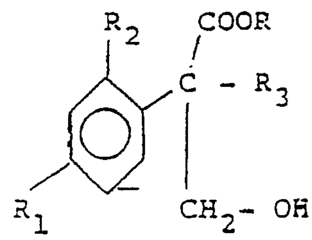 (V)

worin R $CH_3$ oder $C_2H_5$ ist, mit einem Halogenierungs-, Mesylierungs- oder Tosylierungsmittel nach bekannten Verfahren umgesetzt wird, das erhaltene Zwischenprodukt der Formel

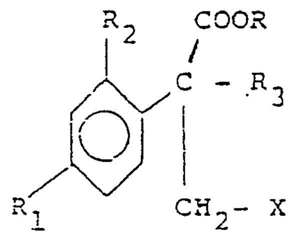 (IV)

worin X Halogen, einen Mesylrest oder einen Tosylrest bedeutet, mit einem Alkalimetallsalz eines Azols der Formel

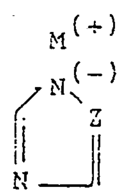

worin M ein Alkalimetall ist und Z die in Anspruch 1 angegebene Bedeutung hat, in einem dipolaren aprotischen Lösungsmittel und bei Temperaturen von 20° C bis zur Rückflußtemperatur der Reaktionsteilnehmer kondensiert wird und die so erhaltene Verbindung der Formel

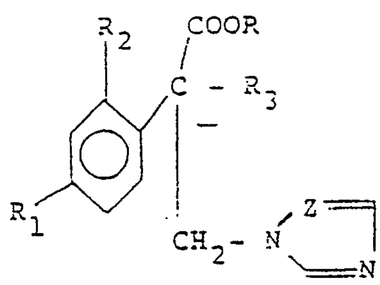 (III)

mit Metallhydriden in Etherlösungsmitteln reduziert wird.

**6.** Verfahren zur Bekämpfung oder Verhütung von Pilzinfektionen bei Nutzpflanzen, das aus dem Verteilen einer wirksamen Menge einer Verbindung gemäß irgendeinem der Ansprüche 1 bis 3 als solcher oder in Form einer geeigneten Zusammensetzung auf die Pflanze, auf die Samen oder die umgebende Erde besteht, wenn der Pilzbefall erwartet wird oder bereits in Gang ist.

**7.** Antifungale Zusammensetzungen, die als Wirkstoff eine oder mehrere Verbindungen gemäß irgendeinem der Ansprüche 1 bis 3 zusammen mit einem festen oder flüssigen Träger und gegebenenfalls anderen Zusätzen enthalten.